## Europäisches Patentamt

## European Patent Office

## Office européen des brevets

(11) Veröffentlichungsnummer: **0 115 062**

**A2**

(12) # EUROPÄISCHE PATENTANMELDUNG

(21) Anmeldenummer: 83113049.7

(22) Anmeldetag: 23.12.83

(51) Int. Cl.³: **G 01 N 33/54**
C 12 N 5/00, C 12 P 21/00

(30) Priorität: 30.12.82 DE 3248617

(43) Veröffentlichungstag der Anmeldung:
08.08.84 Patentblatt 84/32

(84) Benannte Vertragsstaaten:
AT BE CH DE FR GB IT LI LU NL SE

(71) Anmelder: Biotest-Serum-Institut GmbH
Flughafenstrasse 4
D-6000 Frankfurt-Niederrad(DE)

(72) Erfinder: Sonneborn, H.H. Dr.
Birkeneck 70
D-6056 Heusenstamm(DE)

(72) Erfinder: Pfeffer, Almut
Bornemann Strasse 20 I
D-6000 Frankfurt 70(DE)

(72) Erfinder: Schulte, Gertrud
Odenwaldstrasse 32
D-6000 Frankfurt-Niederrad(DE)

(72) Erfinder: Mann, Gisela
Offenbacher Landstrasse 237
D-6000 Frankfurt 70(DE)

(74) Vertreter: Wolff, Hans Joachim, Dr.jur.
Dipl.-Chem. et al,
Beil, Wolff & Beil Rechtsanwälte Postfach 80 01 40
Adelonstrasse 58
D-6230 Frankfurt am Main 80(DE)

(54) Monoklonale Antikörper, im direkten Agglutinationstest reagierend, spezifisch für das humane Blutgruppenantigen D (Rho) und Hybridoma-Zell-Linien, die diese monoklonalen Antikörper produzieren.

(57) Monoklonale Antikörper, im direkten Agglutinationstest, spezifisch für das menschliche Blutgruppenantigen D (Rh$_o$) und Hybridoma-Zell-Linien, die diese monoklonalen Antikörper produzieren, lassen sich dadurch herstellen, daß man Tiere, insbesondere Mäuse mit humanen D-positiven Erythrozyten immunisiert und die von diesen Tieren erhaltenen Milz-Zellen mit Tier- insbesondere Maus-Myeloma-Zellen unter Bildung eines Hybridomas fusioniert. Die Hybridomas werden als Klone gezüchtet und die von den einzelnen Klonen erhaltenen Antikörper auf ihre Spezifität gegen das Blutgruppenmerkmal D in Humanerythrozyten getestet. Klone, die Antikörper produzieren mit einer Spezifität gegen das Blutgruppenmerkmal D von Humanerythrozyten werden für die Weiterzüchtung zur Herstellung des Antikörpers selektiert, wobei der Antikörper aus dem Kulturmedium oder den Ascitesflüssigkeiten des Tieres, insbesondere der Maus, die den Hybridomatumor trägt, gewonnen wird.

EP 0 115 062 A2

Unsere Nr. 24 288                    Pr/br

Biotest-Serum-Institut GmbH
Flughafenstraße 4
6000 Frankfurt 71

Monoklonale Antikörper, im direkten Agglutinationstest reagierend, spezifisch für das humane Blutgruppenantigen D (Rh$_O$) und Hybridoma-Zell-Linien, die diese monoklonalen Antikörper produzieren

Die Erfindung betrifft monoklonale Antikörper, im direkten Agglutinationstest reagierend, spezifisch für das menschliche Blutgruppenantigen D (Rh$_O$), d.h. Antikörper mit Spezifität gegen das Blutgruppenmerkmal D humaner Erythrozyten sowie Hybridoma-Zell-Linien, die diese monoklonalen Antikörper produzieren.

Antikörper werden seit langem in der medizinischen Diagnostik verwendet. Ihre Verwendbarkeit ist jedoch etwas begrenzt, da es aufgrund ihrer Komplexizität und Vielseitigkeit schwierig ist, homogene Antikörper zu erzielen. Antikörper sind komplexe Proteinmoleküle bzw. Moleküle auf Proteinbasis, die durch das Immunsystem von Menschen oder Tieren produziert werden, um den Menschen oder das Tier gegen Antigene zu schützen. Antikörper für die medizinische Verwendung werden im allgemeinen dadurch erhalten, daß man einem Tier ein Antigen injiziert, welches das Immunsystem des Tieres stimuliert, und eine Antikörperfraktion aus dem peripheren Blutserum oder aus der Ascitesflüssigkeit isoliert.

Die Antikörperfraktion enthält Antikörper mit Spezifität gegen das injizierte Antigen sowie zahlreiche andere Antikörper, die von dem Tier produziert wurden. Nach bekannten Verfahren kann es möglich sein, im wesentlichen einen Antikörper zu isolieren mit Spezifität gegen das bestimmte Antigen. Jedoch selbst wenn ein Antikörper für ein bestimmtes Antigen isoliert wird, ist dieser Antikörper meistens ein Gemisch aus verschiedenen Antikörpern, die mit unterschiedlichen antigenen Determinanten des Antigens reagieren können.

Der Versuch, Zellen in vitro dazu zu bringen, in größerer Menge und vor allem über längere Zeit hin spezifische Antikörper zu produzieren, scheiterte daran, daß die Zellen in vitro schnell inaktiviert werden und ihre Antikörperproduktion einstellen.

In den letzten Jahren wurden Verfahren zur Herstellung monoklonaler Antikörper entwickelt, die es möglich machen, homogene hochspezifische Antikörper zu erzielen. Im allgemeinen werden solche Antikörper dadurch hergestellt, daß man ein Tier mit einem Antigen immunisiert, Antikörper produzierende Zellen von diesem Tier erhält und die Antikörper produzierenden Zellen mit Tumor-Zell-Linien, z.B. Myeloma-Zell-Linien, fusioniert, wobei man Hybridomas erhält, die isoliert werden und die die monoklonalen Antikörper produzieren (Köhler, G. und Milstein, C., Eur.J.Immunol., 6, 511-519 (1976) und Köhler, G. und Milstein, C., Nature, 256, 495 (1975).

Die Zellen des Hybridomas haben von dem einen "Elternteil" das Vermögen mitbekommen, einen ganz bestimmten Antikörper zu produzieren. Dem anderen "Elternteil", dem Myelom, verdanken sie ihre Fähigkeit, sich auf lange Zeit hin weiter zu teilen.

Diese Hybridomas können entweder in vitro oder aber als Tumoren in einem Wirtstier gezüchtet werden.

Da jede Antikörper produzierende Zelle (Klon) nur Antikörper einer einzigen Spezifität bildet, produzieren die monoklonalen Kulturen der Hybridomas, die aus einem solchen Klon mit einer Spezifität gezüchtet wurden, jeweils einen homogenen Antikörper, der entweder aus dem Kulturmedium von Hybridoma-Kulturen in vitro oder aus den Zellen, Ascitesflüssigkeiten oder Serum von tumortragenden Wirtstieren erhalten werden kann.

Nicht alle Hybridomaklone, die aus der Fusionierung von neoplastischen Zellen mit Antikörper produzierenden Zellen resultieren, sind spezifisch für das gewünschte Antigen, da viele Hybridomas Antikörper erzeugen, die das immunisierte Tier gegen andere Antigene produziert hat. Selbst Antikörper gegen spezifisches Antigen können von Klon zu Klon unterschiedlich sein, da Antikörper von unterschiedlichen Zellen mit unterschiedlichen anti-genen Determinanten des gleichen Moleküls reagieren können. Deshalb ist es erforderlich, den entstehenden Antikörper oder das die Antikörper enthaltende Medium, Serum oder Ascitesflüssigkeit auf seine Reaktivität mit dem bestimmten Antigen zu testen und seine Spezifität zu testen, indem man bestimmt, mit welchen anderen Antigenen er reagiert.

Aufgrund der Homogenität und hohen Spezifität der auf diese Weise hergestellten monoklonalen Antikörper sind letztere äußerst wertvoll für diagnostische Zwecke.

Monoklonale Antikörper mit Spezifität gegen Blutgruppenmerkmale A und B sind bereits bekannt und lassen sich zur Bestimmung dieser Blutgruppenmerkmale innerhalb der Transfusionsmedizin und für Forensische Untersuchungen verwenden (vgl.

VOAK, D., SACKS, S., ALDERSON, T., TAKEI, F., LENNOX, E.,
JARVIS, J., MILSTEIN, C. und DARNBOROUGH, J. (1980);
Monoclonal Anti-A from a Hybrid Myeloma: Evaluation
as a Blood Grouping Reagent. Vox Sanguinis, 39, 134-140).

Es bestand jedoch ein Bedarf, Antikörper mit Spezifität
gegen andere Blutgruppenmerkmale humaner Erythrozyten herzustellen.

Erfindungsgemäß lassen sich nunmehr monoklonale Antikörper mit Spezifität, im direkten Agglutinationstest
reagierend, gegen das Blutgruppenmerkmal D (Rh$_O$) von
Humanerythrozyten herstellen. Bezüglich der Definition
von D bzw. Rh$_O$ siehe O.Prokop, W. Göhler "Die menschlichen Blutgruppen", Gustav-Fischer-Verlag, New York,
1976, Seiten 73-75).

Um die Produktion von Antikörpern mit Spezifität gegen
das Blutgruppenmerkmal D zu induzieren,
werden Tiere mit humanen D-positiven Erythrozyten
immunisiert. Die dafür ausgewählte Tierspecies ist nicht
kritisch, jedoch sollte der Stamm des Tieres genetisch gut
definiert sein. Da dies für verschiedene Murinstämme,
z.B. Ratten, Mäuse usw., zutrifft und da verschiedene
neoplastische Zellen von Murinen ebenfalls als gut
charakterisierte Kulturen erhältlich sind, wurden
Mäuse für die Herstellung der erfindungsgemäßen Antikörper verwendet. Jedoch lassen sich auch andere Tierspezies für diesen Zweck verwenden.

BALB/c Mäuse werden immunisiert mit humanen D-positiven
Erythrozyten intravenös. Nach der letzten Injektion
werden die Mäuse getötet und die Milzzellen für das
Fusionsexperiment gewonnen.

Danach wird eine Milzzellensuspension hergestellt.
Die Milzzellensuspension wird dann gewaschen, zweckmäßigerweise durch Zentrifugieren in Hank's Medium.
Die so erhaltenen Antikörper
produzierenden Zellen werden mit neoplastischen Zellen
fusioniert. Einige Tumorzellen, insbesondere Myeloma-
Zellen, lassen sich vorteilhafterweise mit den Antikörper
produzierenden Zellen fusionieren und liefern lebensfähige,
Antikörper produzierende Kulturen von Hybridomas. Vorzugsweise sollten die Tumorzellen und die Antikörper produzierenden Zellen von der gleichen Species stammen, da
dabei die Wahrscheinlichkeit, daß die genetischen und
biochemischen Eigenschaften der Elternzellen verträglich
sind, größer wird und dadurch lebensfähige Hybridomas
produziert werden.

Es gibt eine Vielzahl gut charakterisierter Myeloma-
Zell-Linien, die sich erfindungsgemäß verwenden lassen.
Beispiele hierfür sind P3-X63-Ag8, P3-X63-Ag8.653, S 194,
Y3, SP2/0, MPC-11 und deren Derivate. Vorzugsweise wird
die Linie P3-X63-Ag8.653 (CNCM I -251) (Kearney et al,
J.Immunol.123:1548(1979) verwendet. Vorzugsweise
wählt man eine Myeloma-Linie aus, die keinen Antikörper
produziert, so daß das dabei entstehende Hybridoma nur
Antikörperketten von der Eltern-Milz- oder -Lymphknoten-
Zelle produziert.

Die Myeloma-Zellen werden zweckmäßigerweise in RPMI 1640'
gehalten, dem 10 % FCS zugesetzt werden. Myeloma-Zellen
und Zellen, die von Mäusen erhalten wurden, die mit
humanen D-positiven Erythrozyten immunisiert worden
waren, werden nach der Methode von Köhler, G. und
Milstein, C., Eur.J.Immunol.6, 292 (1976) fusioniert.

Die Hybridomas werden durch die Verwendung von Hypoxanthin-Aminopterin-thymidin (HAT) Medium selektiert, wobei jegliches Wachstum in HAT-Medium die erfolgreiche Hybridisierung von Maus-Milz-Zellen und Maus-Myloma-Zellen anzeigt. Ihre Produktion von Antikörpern gegen die zur Immunisierung der Maus verwendeten D-positiven Erythrozyten wird mit Hilfe von Haemagglutinationstests festgestellt. Hybridzellen werden mit Hilfe der "limiting dilution method" in Mikrotiterplatten kloniert.

Klone von Hybridomas können in vitro gezüchtet werden nach den bekannten Gewebekulturmethoden, wie beispielsweise durch Cotton et al., Eur.J.Immunol. 3, 136 (1973) beschrieben. Wahlweise lassen sich die Hybridomas jedoch auch in vivo als Tumoren in einem histokompatiblen Tier oder in athymischen Nacktmäusen züchten. Die Antikörper lassen sich aus dem in vitro Kulturmedium oder aus dem Serum oder der Ascitesflüssigkeit des Tieres nach bekannten Methoden gewinnen.

Die Spezifität des Antikörpers von jedem Klon gegen das Blutgruppenmerkmal D in Humanerythrozyten wird mit Hilfe von Haemagglutinationstests festgestellt und Klone, die Antikörper produzieren mit Spezifität gegen das Blutgruppenmerkmal D in Humanerythrozyten, werden selektiert.

Beispiel

Herstellung von Hybridoma-Zell-Linien

BALB/c Mäuse wurden immunisiert mit $5 \times 10^8$ humanen D-positiven Erythrozyten intravenös, 2 x wöchentlich mit insgesamt 10 Injektionen. 4 Tage nach der letzten Injektion wurden die Mäuse getötet und die Milzzellen für das Fusionsexperiment gewonnen.

Danach wurde eine Milzellensuspension hergestellt. die Milzzellensuspension wurde 2 x durch Zentrifugieren (800 x g) in Hank's Medium gewaschen.

Hybridoma-Zell-Linien wurden erhalten durch Fusion von $10^8$ der auf vorstehende Weise gewonnenen Milzzellen mit $10^7$ Mausmyeloma-Zellen P3-X63-Ag8.653(CNCM I-251), die in RPMI 1640 gehalten wurden, dem 10 % FCS zugesetzt wurde.

Die Zellmischung wurde bei 800 x g 2 x zentrifugiert, resuspendiert zur Fusion in 40 % (v/v)iger Polyethylen-glykol (PEC) 4000-Lösung in Hank's Medium. Die Hybridomas wurden durch die Verwendung von HAT-Medium auf folgende Weise selektiert und auf Antikörper mit Spezifität gegen das Blutgruppenmerkmal D untersucht.

Es wurden 570 Näpfe einer Mikrotiterplatte, gefüllt mit 200 µl Zellsuspension und einer Zelldichte von 1,3 x $10^5$ Zellen/ml, ausgesät. Nach 12 Tagen Inkubation bei 37°C und 5 % $CO_2$ wurden in 224 Näpfen Klone gefunden.

Von den 224 Näpfen wurden von 174 Näpfen Überstände auf Antikörper gegen Blutgruppenantigene untersucht.

Die Überstände wurden im direkten Agglutinationstest mit dem bekannten Röhrchenzentrifugationstest untersucht.

Einer dieser Überstände zeigte eine Spezifität für das Merkmal D.

Dieser Napf wurde nach der "limiting dilution" Methode kloniert. Insgesamt wurden 576 Näpfe ausgesät. 152 davon zeigten Klonwachstum, in 21 Näpfen davon wuchs nur 1 Klon. Diese 21 Näpfe wurden auf Spezifität getestet: 19 von 21 waren positiv für das Merkmal D.

Die Tabelle zeigt eine typische Reaktion der erhaltenen Hybridomaüberstände mit verschiedenen Testzellen.

## Tabelle

| Antikörper-Identifizierungstabelle | | | | | | | | | Stufe | | | Zusatz-tests | | Kell | | | | Duffy | | Luth-eran | | Kidd | | MNS | | | | Lewis | | P | Xg | Colton | | Dom-brock | | | Special Type |
|---|---|---|---|---|---|---|---|---|---|---|---|---|---|---|---|---|---|---|---|---|---|---|---|---|---|---|---|---|---|---|---|---|---|---|---|---|---|
| Blutgruppensystem | | | Rh-hr | | | | | | Zentrifug.-Test | 37° | Coombs | | | K | k | Kpᵃ | Kpᵇ | Fyᵃ | Fyᵇ | Luᵃ | Luᵇ | Jkᵃ | Jkᵇ | M | N | S | s | Leᵃ | Leᵇ | P1 | Xgᵃ | Coᵃ | Coᵇ | Doᵃ | Doᵇ | | |
| Donor No. | Rh-Typ | | D | C | E | c | e | Cʷ | I | II | III | | | | | | | | | | | | | | | | | | | | | | | | | | |
| 107 675 | R₁R₁ | 1 | + | + | O | O | + | O | 3 s | | | | | O | + | + | + | O | + | O | + | O | + | + | + | O | + | + | O | + | + | + | O | + | / | 1 | B7 Bg (a+) |
| 319 314 | R₂R₂ | 2 | + | O | + | + | O | O | 3 s | | | | | O | + | O | + | O | + | O | + | + | + | + | + | + | + | O | + | + | + | / | O | / | / | 2 | |
| 301 146 | R⁻R₁ | 3 | + | + | O | O | + | + | 3 w | | | | | O | + | O | + | + | + | O | + | + | O | + | O | + | O | + | O | + | + | + | O | O | / | 3 | |
| 301 159 | R⁻r | 4 | + | O | O | + | + | O | 3 w | | | | | O | + | O | + | + | + | + | + | O | + | O | + | + | + | O | + | O | + | + | O | O | / | 4 | |
| 122 600 | r'r | 5 | O | + | O | + | + | O | – | | | | | + | O | O | + | + | O | O | + | + | + | + | O | + | O | O | + | O | + | + | O | O | / | 5 | A 28  Bg (c+) Rd+ |
| 121 943 | r''r | 6 | O | O | + | + | + | O | – | | | | | O | + | O | + | + | O | O | + | + | + | O | + | O | + | O | + | + | O | + | O | + | / | 6 | |
| 120 325 | rr | 7 | O | O | O | + | + | O | – | | | | | + | + | O | + | + | O | + | O | + | + | + | O | O | + | O | O | + | + | + | O | + | / | 7 | |
| 320 228 | rr | 8 | O | O | O | + | + | O | – | | | | | O | + | O | + | + | + | O | + | O | + | + | O | + | O | + | O | + | + | + | O | / | / | 8 | |

+ = positiv   O = negativ   / = nicht bestimmt   , w = schwach   a = stark

Agglutinationsstärke

der positiven

Überstände (1 = schwach; 4 = stark; – = negativ)

- 10 -

Hybridomas, die Antikörper mit dem in der Tabelle angegebenen Reaktionsmuster zeigten, wurden mit der
"Limiting dilution method" kloniert.

So erhaltene Hybridoma -Linien wurden etabliert und sowohl in Überständen als auch in Ascitesflüssigkeiten,
die nach Inocculation der Hybridoma-Linien in BALB/c-
Mäusen gewonnen wurden, auf Spezifität und Avidität
mit Test-Erythrozyten untersucht.

Die erhaltene Hybridoma-Linie ist unter der Nr. BS46
(CNCM I-269) etabliert und hinterlegt.

Die Hybridoma-Linie BS 46 (CNCM I-269) produziert einen Antikörper der
Klasse IgG 1.Die Linie BS 46 (CNCM I-269)produziert diesen Antikörper permanent in Zellsuspensionskulturen in vorzugsweise RPMI 1640-Medium.

Die Hybridoma-Linie BS46 (CNCM I-269)wächst auch als intraperitonealer
Tumor in BALB/c-Mäusen und produziert unter diesen
Bedingungen IgG 1 Antikörper mit der beschriebenen
Spezifität, die aus dem Ascites der tumortragenden
Tiere gewonnen werden können.

Die erfindungsgemäßen Antikörper sind wertvolle Hilfsmittel für die Transfusionsmedizin und für Forensische
Untersuchungen.

Patentansprüche

1. Hybridoma-Zell-Linie, die einen Antikörper mit Spezifität gegen ein Blutgruppenmerkmal von humanen Erythrozyten produziert, erhalten durch Fusion einer Antikörper produzierenden Tierzelle und einer neoplastischen Zelle, d a d u r c h  g e k e n n z e i c h n e t, daß sie einen Antikörper mit Spezifität, im direkten Agglutinationstest reagierend, gegen das Blutgruppen merkmal D humaner Erythrozyten produziert.

2. Zell-Linie nach Anspruch 1, dadurch gekennzeichnet, daß die Antikörper produzierende Zelle murinen Ursprungs ist.

3. Zell-Linie nach einem der vorstehenden Ansprüche, dadurch gekennzeichnet, daß die Antikörper produzierenden Zellen BALB/c Maus-Milzzellen sind.

4. Zell-Linie nach einem der vorstehenden Ansprüche, dadurch gekennzeichnet, daß die neoplastische Zelle eine Myeloma-Zelle ist.

5. Zell-Linie nach einem der vorstehenden Ansprüche, dadurch gekennzeichnet, daß die neoplastische Zelle die Myeloma-Zell-Linie P3-X63-Ag8.653 (CNCM I-251) ist, die keine Antikörper produziert.

6. Hybridoma-Zell-Linie BS 46 (CNCM I-269).

7. Monoklonaler Antikörper, gekennzeichnet durch seine Spezifität gegen das Blutgruppenmerkmal D humaner Erythrozyten im direkten Agglutinationstest reagierend.

8. Monoklonaler Antikörper nach Anspruch 7, dadurch gekennzeichnet, daß er von einer Hybridoma-Zell-Linie gemäß Anspruch 1 bis 6 produziert worden ist.

9. Verwendung des Antikörpers nach Anspruch 7 oder 8 zur Bestimmung des Blutgruppenmerkmals D humaner Erythrozyten.